Europäisches Patentamt

European Patent Office

Office européen des brevets

(19)

(11) **EP 1 237 475 B1**

(12) **EUROPÄISCHE PATENTSCHRIFT**

(45) Veröffentlichungstag und Bekanntmachung des Hinweises auf die Patenterteilung:
**06.05.2004 Patentblatt 2004/19**

(21) Anmeldenummer: **00993374.8**

(22) Anmeldetag: **11.12.2000**

(51) Int Cl.$^7$: **A61B 5/0484**, A61B 5/0496

(86) Internationale Anmeldenummer:
**PCT/DE2000/004395**

(87) Internationale Veröffentlichungsnummer:
**WO 2001/043637 (21.06.2001 Gazette 2001/25)**

(54) **VERFAHREN UND ANORDNUNG ZUR BESTIMMUNG DER TOPOGRAPHIE FÜR REAKTIONSSIGNALE EINES AUGES**

METHOD AND SYSTEM FOR DETERMINING THE TOPOGRAPHY FOR REACTION SIGNALS OF AN EYE

PROCEDE ET SYSTEME POUR DETERMINER LA TOPOGRAPHIE DE SIGNAUX DE REACTION DE L'OEIL

(84) Benannte Vertragsstaaten:
**AT BE CH CY DE DK ES FI FR GB GR IE IT LI LU MC NL PT SE TR**

(30) Priorität: **13.12.1999 DE 19959881**

(43) Veröffentlichungstag der Anmeldung:
**11.09.2002 Patentblatt 2002/37**

(73) Patentinhaber: **Medizin & Service GmbH**
**09577 Niederwiesa (DE)**

(72) Erfinder:
• **KUTSCHBACH, Ernst**
**09119 Chemnitz (DE)**
• **GRÜNEWALD, Jens**
**09212 Limbach-Oberfrohna (DE)**

(74) Vertreter: **Seerig & Hübner**
**Patentanwälte**
**Am Alten Bad 6**
**09111 Chemnitz (DE)**

(56) Entgegenhaltungen:
**DE-A- 19 649 858**

• **SPARKS S ET AL: "COMPARISON OF REPEATABILITY OF THE MULTIFOCAL ELECTRORETINOGRAM AND HUMPHREY PERIMETER" DOCUMENTA OPHTHALMOLOGICA,NL,KLUWER, DORDRECHT, Bd. 92, 1997, Seiten 281-289, XP002062777**
• **JIANG X ET AL: "MEASUREMENT OF THE MICRO-ELECTRORETINOGRAM AND COMPONENT ANALYSIS" MEDICAL AND BIOLOGICAL ENGINEERING AND COMPUTING, PETER PEREGRINUS LTD. STEVENAGE, GB, Bd. 31, 1. Juli 1993 (1993-07-01), Seiten S73-79, XP000399681 ISSN: 0140-0118**
• **FADDA A ET AL: "INSTRUMENTATION FOR ELECTRORETINOGRAPHY: A LED-BASED STIMULATOR" ANNUAL INTERNATIONAL CONFERENCE OF THE IEEE ENGINEERING IN MEDICINE AND BIOLOGY SOCIETY,US,NEW-YORK, NY: IEEE, 31. Oktober 1996 (1996-10-31), Seiten 152-153, XP000787630 ISBN: 0-7803-3812-X**
• **PATENT ABSTRACTS OF JAPAN vol. 016, no. 140 (C-0926), 8. April 1992 (1992-04-08) & JP 03 297443 A (ULVAC JAPAN LTD), 27. Dezember 1991 (1991-12-27)**

**Beschreibung**

Die Erfindung betrifft ein Verfahren und eine Anordnung zur nicht-invasiven Bestimmung der Topographie für Reaktionssignale eines Auges, wobei Teilflächen der Netzhaut zyklisch gereizt werden und dabei die Gesamtreaktion des Auges im eingeschwungenen Zustand gemessen wird.

Die ermittelte Topographie für die Reaktionssignale eines Auges zeigt die objektive Empfindlichkeitsverteilung der Netzhaut und gibt somit Aufschluß über das Sehvermögen. Mit einer solchen Untersuchung ist eine Früherkennung und Bewertung von Augenkrankheiten, z.B. des Glaukom, möglich. Vor allem sind mit derartigen Untersuchungen partielle Fehler der Netzhaut feststellbar.

Es gibt eine Vielzahl von Untersuchungsmethoden, die auf subjektiver Basis arbeiten, d.h. bei denen der untersuchte Patient die Messung durch seine Aussage bewertet. Bei allen diesen Methoden ist der Patient in die Messung integriert, indem er die Aussage gibt, ob und wie er einen bestimmten Reiz wahrnimmt.

Als objektives Meßverfahren hat sich das Elektroretinogramm (ERG) etabliert, bei dem das mit einer Elektrode vom Auge abgenommene Reaktionssignal mit seinem zeitlichen Verlauf dargestellt und ausgewertet wird. Zur Stimulierung werden einmalige Lichtblitze oder zyklische Hell-Dunkelfolgen (Flicker-ERG) verwendet. Dabei wird der Mittelwert für die gesamte Netzhautfläche bestimmt. Bei der Aufnahme evozierter Potentiale werden Elektroden an bestimmter Stelle des Kopfes angebracht, und das gemessene Signal entspricht der Reaktion, die über Nervenstränge an der Meßposition auftritt. Einzelheiten hierzu sind von J. Jörg und H. Hielscher in dem Buch "Evozierte Potentiale in Klinik und Praxis", Springer Verlag, 3.Auflage, veröffentlicht.

Zur Bestimmung der Topographie der Netzhautempfindlichkeit besteht grundsätzlich die Möglichkeit, Teilflächen der Netzhaut durch einzelne, der jeweiligen Teilfläche zugeordnete, Lichtreize zu stimulieren und die Reaktion zu messen. Da zur Reduzierung des Rauschens Mittelwerte über eine Vielzahl von Messungen gebildet werden müssen, ergibt sich bei diesem Verfahren eine unvertretbar lange Meßzeit.

In der Patentschrift US 5 382 987 wird die Kopplung einer ophthalmoskopischen Anordnung auf Basis eines 3-Wege Maxwell Betrachtungssystems zur optischen Untersuchung der Netzhaut des Auges mit einer perimetrischen Anordnung zur Bestimmung des Gesichtsfeldes vorgeschlagen, wobei die spektrale Empfindlichkeit eines ausgewählten Teiles der Netzhaut mit Hilfe des Elektroretinogrammes gemessen werden kann, für das ein Stimulusmuster auf die Netzhaut übertragen wird. Mit diesem Verfahren kann die Empfindlichkeit eines einzelnes Teiles sowie das Gesamtbild der Netzhaut untersucht werden. Für die meßtechnische Untersuchung aller Teile der Netzhaut würde sich auch hier eine unvertretbar lange Untersuchungszeit ergeben.

Die Verwendung eines mit einem optischen Modulator versehenen Laserprojektors zur Erzeugung eines Helligkeitsmusters auf der Netzhaut für die Messung des Muster-Elektroretinogramms (PERG) ist von Daniel R. Peters und John Tabora in der Patentschrift US 5 233 373 angegeben worden. Damit kann aber auch nur immer eine ausgewählte Fläche der Netzhaut untersucht werden, und die Untersuchung mehrerer Flächen kann nur nacheinander erfolgen.

Ein verbessertes Verfahren und die zugehörige Anordnung zur Bestimmung der Funktionsverteilung der Reaktion über die Fläche der Netzhaut auf Reize ist von R. Richardson in der Patentschrift EP 0 375 737 angegeben worden. Hierbei wird die Gesamtreaktion der Netzhaut auf Stimuli im Sehfeld aufgenommen, wobei die Stimuli durch Serien von Mustern gebildet werden, deren Intensität sich in horizontaler Richtung und in vertikaler Richtung ändert. Als Beispiel wird eine sinus- bzw. cosinusförmige Verteilung der Intensität verwendet, und durch Rücktransformation aus den gemessenen Gesamtsignalen läßt sich die Empfindlichkeitsverteilung über die Fläche der Netzhaut berechnen. Nachteilig bei diesem Verfahren ist, daß sich Meßfehler bei der Bestimmung der einzelnen Koeffizienten nicht erkennen lassen, daß sich aber einzelne Meßfehler auf die Berechnung der gesamten Verteilungsfunktion auswirken. Nachteilig ist bei diesem Verfahren weiterhin, daß die Auflösung auf die größte Dichte der Empfindlichkeitsverteilung angepaßt werden muß, obwohl diese nur in einem eng begrenzten Bereich vorhanden ist.

In der Patentschrift US 5 539 482 wird ein Verfahren angegeben, bei dem das Stimulusbild aus mehreren Vierecken mit vom Zentrum nach außen zunehmender Größe besteht, deren Helligkeitsverlauf mit unterschiedlichen Frequenzen im Bereich von 10 Hz bis 45 Hz gesteuert wird. In dem dargestellten Beispiel wird mit 9 gleichzeitig in der Helligkeit modulierten Vierecken gearbeitet, und die Auswertung des gemessenen Signals erfolgt mit Hilfe der Fouriertransformation. Dabei können zwar durch Messung der Nyquistfrequenz die Einflüsse der unteren Ganglienzellschichten erfaßt werden, aber zur Bestimmung der Nyquistfrequenz müssen mehrere Messungen mit unterschiedlicher Verteilung der Modulationsfrequenzen durchgeführt werden, und die Meßzeit muß für jede Frequenzverteilung so lang gewählt werden, daß eindeutige Ergebnisse für die einzelnen Frequenzen ermittelt werden können. Es wird angegeben, daß eine Erweiterung bis auf 32 "Zonen" möglich ist. Damit hat das Verfahren selbst bei dieser Erweiterung noch eine sehr niedrige Auflösung hinsichtlich der untersuchbaren Teilflächen.

Ein anderes Verfahren wurde von E. E. Sutter und D. Tran in der Zeitschrift Vision Research (Great Britain) Vol. 32, No. 3, pp 433-446, 1992 (E.E. Sutter et al.: "The Field Topography of ERG Components in Man") angegeben, mit dem relativ gute Ergebnisse erzielt worden sind. Zur Bestimmung der Topographie der ERG Komponenten wird ein digitales Verfahren angewendet, bei dem als Stimuli Sechsecke verwendet werden, deren zeitliche Helligkeitsverläufe durch m-Sequenzen gesteuert werden. Dabei werden 241 Sechsecke verwendet, deren Größe vom Mittelpunkt aus nach außen zunimmt. Damit wird der ungleichen Dichte der Verteilungsfunktion Rechnung getragen. Es wird das Gesamtreaktionssignal des Auges gemessen, und durch Berechnung der Kreuzkorrelationsfunktion mit der jeweiligen m-Sequenz wird der Signalverlauf für das betreffende Sechseck berechnet. Durch Wichtung des Signalverlaufes mit dem Mittelwert für den entsprechenden Bereich von Teilflächen werden Störungen bei der Bestimmung der in dem Signalverlauf enthaltenen Amplitude des Nutzsignales reduziert. Für die Messung werden m-Sequenzen mit einer Länge von 65535 Schritten verwendet,

die gegeneinander immer um 256 Schritte versetzt sind. Bei der Bildwechselfrequenz von 67 Hz ergibt sich eine Gesamtmeßdauer von etwa 16 Minuten, wobei die Messung in 32 zeitliche Abschnitte unterteilt wurde, die jeweils 30 Sekunden, zuzüglich einer Zeit für die Überlappung, betragen. Ein erster Nachteil ist, daß das Verfahren nur in dem Umfang exakte Ergebnisse liefern kann, wie die Reaktionsfunktion der Netzhaut auch linear auf die Reize reagiert. Dieser Zusammenhang ist aber bei einer Bildwiederholfrequenz von 67 Hz nur teilweise, und dann auch nur bei kurzer Nachleuchtdauer des Bildschirmes, gegeben. Ein weiterer Nachteil dieses Verfahrens besteht darin, daß der Signalverlauf subjektiv überwacht werden muß und bei erkennbaren Störungen, z.B. durch Lidschlag oder bei Kontaktproblemen der Elektrode, der zugehörige Zeitabschnitt zu wiederholen ist. Der wesentlichste Nachteil ist aber, daß es keine Möglichkeit gibt, die Zwischenergebnisse der einzelnen Zeitabschnitte zu beurteilen und daß erst am Ende der Messung, d.h. nach Vorliegen der Meßwerte über alle Zeitabschnitte, die Möglichkeit besteht, ein Ergebnis zu ermitteln und dieses Ergebnis zu bewerten, so daß bei nicht erkannten Störungen die gesamte Messung zu wiederholen ist.

In der amerikanischen Patentschrift US 4 846 567 wurde von E. E. Sutter bereits ein Grundprinzip des Verfahrens angegeben, bei dem als Stimuli ein Display mit einem quadratischen Array von aktivierbaren Elementen verwendet wird, wobei die zeitlichen Helligkeitsverläufe der Elemente von m-Sequenzen gesteuert werden. Die Berechnung der einzelnen Reaktionssignale erfolgt mit Hilfe der Kreuzkorrelationsfunktion. Auch hier werden m-Sequenzen der Länge $2^{16} - 1 = 65535$ angewendet, die jeweils um 256 Schritte gegeneinander versetzt sind. Ebenso wird vorgeschlagen, die gesamte Messung in zeitliche Abschnitte von etwa 20 bis 40 Sekunden zu unterteilen.

Eine Weiterentwicklung dieses Verfahrens ist in der Patentschrift DE 196 49 858 C2 angegeben. Auf eine vor dem Auge angeordneten Fläche wird ein aus Teilflächen bestehendes leuchtendes Bild erzeugt, wobei für die Hell- und Dunkelsteuerung der Teilflächen in jedem zeitlichen Abschnitt vollständige verlängerte m-Sequenzen verwendet werden. Aus dem Reaktionssignal des Auges werden mit Hilfe der Kreuzkorrelationsfunktion die Reaktionsfunktionen der Teilflächen in jedem zeitlichen Abschnitt berechnet und durch Bildung von Summenfunktionen wird die Qualität der Ergebnisse für jeden zeitlichen Abschnitt bewertet. Es werden nur mit gut bewertete Reaktionssignale für das Gesamtergebnis aufsummiert. Das Reaktionssignal des Auges wird auf die Einhaltung von Grenzwerten überwacht und bei erkannten Fehlern werden die m-Sequenzen um eine vorgebbare Anzahl von Schritten zurückgesetzt, erkannte Fehler der Reaktionssignale werden durch die Wiederholung ersetzt. Jedes Bild wird in einem ersten einstellbaren Teilschritt angezeigt und während eines zweiten einstellbaren Teilschrittes dunkelgesteuert. Es werden also kurze und korrigierte m-Sequenzen für die Steuerung der Hell-Dunkel-Folgen verwendet. Es sind die einzelnen Zwischenergebnisse bewertbar, und es ist sowohl eine höhere Effektivität erreichbar, als auch eine höhere Sicherheit bei der Bewertung der Ergebnisse möglich.

Die Ermittlung der Topographie der Netzhaut des Auges aus den Reaktionssignalen, die sich bei zyklischer Reizung im eingeschwungenen Zustand ergeben, ist sowohl mit den von E. E. Sutter als auch mit dem in dem Patent DE 196 49 858 C2 angegebenen Verfahren möglich, hat aber den Nachteil, daß für gleiche Zeitabschnitte ein schlechteres Signal-Rausch-Verhältnis entsteht, da bei Verwendung von m-Sequenzen nur jeder zweite Schritt des Meßablaufes ein Nutzsignal erzeugt, aber in jedem Schritt der m-Sequenz entsteht Rauschen und zusätzlich muß in jedem Schritt der m-Sequenz erst der eingeschwungene Zustand hergestellt werden, bevor eine Auswertung erfolgen kann. Es kann also auch nur ein Teil der Reaktionssignale eines Schrittes ausgewertet werden, und das Ergebnis in diesem Teil wird durch zwei mal Rauschen und ein mal Nutzsignal gebildet.

Der Erfindung liegt die Aufgabe zugrunde, ein Verfahren und eine Anordnung zur nicht-invasiven Bestimmung der Topographie für Reaktionssignale eines Auges zu schaffen, mit der die Topographie der Netzhautreaktionen bei zyklischer Reizung im eingeschwungenen Zustand im gleichen Zeitraum mit besserem Signal-Rausch-Abstand oder bei vergleichbarem Signal-Rausch-Abstand in einem wesentlich kürzeren Zeitraum ermittelt werden kann.

Erfindungsgemäß wird die Aufgabe dadurch gelöst, daß bei der Messung das Reaktionssignal jeder Teilfläche über die gesamte Dauer der Messung genutzt werden kann, indem jede Teilfläche durch eine ganzzahlige Anzahl von Schwingungen stimuliert wird und daß sich die Anzahl der Schwingungen für die einzelnen Teilflächen jeweils um ein ganzzahliges Vielfaches von 1 unterscheidet. Dabei wird in einem vorgegebenen Zeitabschnitt für die Messung eine Taktfolge mit einer bestimmten Gesamtanzahl von Takten erzeugt aus der eine ganzzahlige Anzahl von Zyklen für jeweils eine Schwingung erzeugt wird. Die Erzeugung der Schwingungen für die Teilflächen erfolgt mit den folgenden drei Wegen:

- Für eine erste Teilfläche wird eine mittlere Anzahl von Schwingungen gebildet, indem die in der Taktfolge erzeugten Zyklen verwendet werden
- Für eine erste Gruppe von Teilflächen wird eine erste Gruppe von Schwingungen gebildet, indem die in der Taktfolge erzeugten Schwingungen in bestimmten und weitgehend konstanten Abständen jeweils um einzelne Takte verlängert werden, wodurch sich eine kleinere Anzahl von Schwingungen in der vorgegebenen Gesamtanzahl von Takten ergibt.
- Für eine zweite Gruppe von Teilflächen wird eine zweite Gruppe von Schwingungen gebildet, indem die in der Taktfolge erzeugten Schwingungen in bestimmten und weitgehend konstanten Abständen jeweils um einzelne Takte verkürzt werden, wodurch sich eine größere Anzahl von Schwingungen in der vorgegebenen Gesamtanzahl von Takten ergibt.

Innerhalb jeder Gruppe wird für die Teilflächen ein anderer Abstand für die Verlängerung oder Verkürzung um einen Takt verwendet und die Anzahl dieser Takte ist jeweils ein ganzzahliges Vielfaches der Anzahl der für die mittlere Schwingung verwendeten Takte. Für das Erreichen des eingeschwungenen Zustandes werden mehrere zusätzliche Schwingungen vor dem Zeitabschnitt für die Messung und für die Auswertung der letzten Schwingung wird wenigstens eine zusätzliche Schwingung nach dem Zeitabschnitt für die Messung vorgesehen. Das aus der Überlagerung der Reaktionssignale aller Teilflächen gebildetes Summensignal wird nicht-invasiv am Patienten abgenommen, wird verstärkt und gefiltert und einem Analog-Digital-Wandler zugeführt, der das Signal mit den gleichen Takten übernimmt, die für die Erzeugung der Schwingungen verwendet werden. Die Rückgewinnung der Reaktionssignale für die einzelnen Teilflächen erfolgt durch zyklische Summation der aufeinanderfolgenden Teile des Summensignals, wobei für die Summation dieselben Zyklen verwendet werden, wie für die Schwingungen zur Stimulierung der entsprechenden Teilflächen.

Die Anordnung für die Durchführung des erfindungsgemäßen Verfahrens wird durch die in Anspruch 8 genannten Merkmale gelöst. Vorteilhafte Ausgestaltungen sind in den Unteransprüchen angegeben.

Der Vorteil der erfindungsgemäßen Lösung besteht darin, daß es eine objektive Meßmethode ist, d.h. die Messung erfolgt unabhängig davon ob und wie der Mensch welchen Reiz wahrnimmt. Ein weiterer Vorteil der erfindungsgemäßen Lösung ist die hohe Auflösung hinsichtlich der zu untersuchenden Teilflächen der Netzhaut, die gleichzeitig untersucht werden, wodurch sich eine kurze Meßzeit ergibt und die Möglichkeit der Bewertung von Zwischenergebnissen, wodurch eine hohe Sicherheit für die Zwischenergebnisse erreicht wird. Darüber hinaus hat die erfindungsgemäße Lösung den Vorteil, daß bei der Messung im eingeschwungenen Zustand (steady-state-Signale) der eingeschwungene Zustand über die gesamte Meßzeit erhalten bleibt und insbesondere, daß von allen Teilschritten des Meßablaufes ein Nutzsignal erzeugt wird, wodurch sich ein wesentlich besseres Signal/Rausch Verhältnis ergibt.

Die erfindungsgemäße Lösung soll anhand eines Ausführungsbeispieles näher erläutert werden. In der dazugehörigen Zeichnung zeigen:

Fig. 1    den Prinzipaufbau der erfindungsgemäßen Lösung,
Fig. 2    ein Zeitdiagramm für die Erzeugung der Schwingungen,
Fig. 3    ein Diagramm zum Prinzip der zyklischen Addition,
Fig. 4    ein Zeitdiagramm für die zyklische Addition,
Fig. 5    eine Tabelle mit praktischen Werten der Schwingungen und
Fig. 6    eine LED - Fläche mit einem Bild aus 61 Sechsecken.

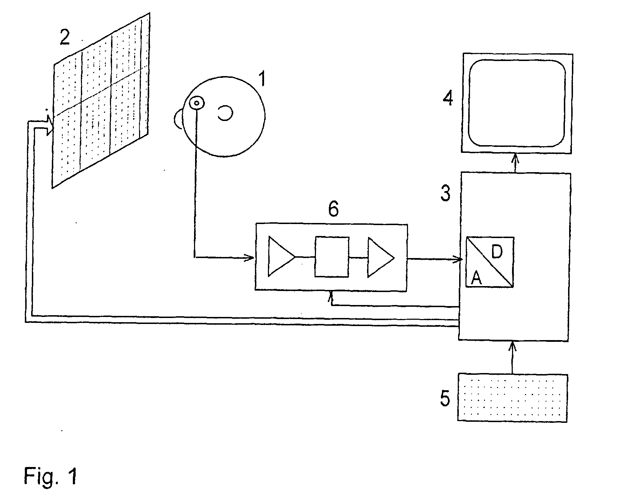

In Fig. 1 ist der Prinzipaufbau der für die Messung verwendeten Anordnung dargestellt. Ein Patient 1 blickt auf einen Stimulator 2, der ein leuchtendes Bild erzeugt, das auf der Netzhaut des Auges abgebildet wird. Die Hell- und Dunkelsteuerung der Teilflächen des Stimulators 2 erfolgt über eine Steuereinheit 3. Die Bedienung der Anordnung, d.h. Einstellung der Meßparameter und der Auswertemethoden erfolgt über eine mit der Steuereinheit 3 verbundene Tastatur 5. Über eine mit der Steuereinheit 3 verbundene Anzeigeeinheit 4 erfolgt die Bedienerführung und die Auswertung der Ergebnisse. Das am Patienten 1 nicht-invasiv abgenommene Reaktionssignal wird über einen Verstärker 6 dem in der Steuereinheit 3 angeordneten Analog-Digital-Wandler 7 zugeführt. Der Verstärker 6 enthält mehrere Verstärkerstufen und einen Bandpaß und ist über die Steuereinheit 3 in seinen Parametern programmierbar.

Um die einzelnen Teilflächen des Stimulators 2 zyklisch hell und dunkel zu steuern werden Schwingungen verwendet, wobei in einem vorgegebenen Zeitraum für jede Teilfläche eine andere Anzahl von Schwingungen verwendet wird.

Ein einfaches Zahlenbeispiel verdeutlicht das Prinzip: Geht man von einer 30 Hz Flicker Stimulierung aus, dann ergeben sich für eine Zeitdauer von 10 Sekunden $N_1 = 300$ Schwingungen. Diese Stimulierung wird für die erste Teilfläche verwendet. Eine zweite Teilfläche erhält in dem gleichen Zeitraum $N_2 = 299$ Schwingungen und eine dritte Teilfläche erhält $N_3 = 301$ Schwingungen. Mit diese Werten ergeben sich die folgenden Frequenzen: $f_1 = 30$ Hz, $f_2 = 29,9$ Hz und $f_3 = 30,1$ Hz. Jede Teilfläche i der Netzhaut erzeugt ein Reaktionssignal r, das aus der Grundwelle der Stimulation und entsprechenden Oberwellen besteht:

$$r_1 = r_{11} \cdot \sin(2\pi \cdot 30\text{Hz} \cdot t) + r_{12} \cdot \sin(2\pi \cdot 60\text{Hz} \cdot t) + ....$$

$$r_2 = r_{21} \cdot \sin(2\pi \cdot 29{,}9\text{Hz} \cdot t) + r_{22} \cdot \sin(2\pi \cdot 59{,}8\text{Hz}\cdot t) + ....$$

$$r_{31} = r_{31} \cdot \sin(2\pi \cdot 30{,}1\text{Hz} \cdot t) + r_{32} \cdot \sin(2\pi \cdot 60{,}2\text{Hz} \cdot t) + ....$$

Das gemessene Signal entspricht der Summe der einzelnen Komponenten. Teilt man nun das über 10 Sekunden gemessene Reaktionssignal in 300 Teile ein und bildet die zyklische Summe dieser Teile, dann erhält man das Reaktionssignal der ersten Teilfläche:

$$R_1 = 300 \cdot r_1 = 300 \cdot \left( r_{11} \cdot \sin(2\pi \cdot 30\text{Hz} \cdot t) + r_{21} \cdot \sin(2\pi \cdot 60\text{Hz} \cdot t) + .... \right)$$

Die anderen Komponenten erzeugen bei der zyklischen Addition kein Signal, da deren Grundwelle und alle Oberwellen immer mit einer anderen Phasenlage über eine oder mehrere volle Schwingungen addiert werden.
Analog erhält man bei einer Einteilung des über 10 Sekunden gemessenen Signals in 299 Teile das Reaktionssignal der zweiten Teilfläche und bei einer Einteilung in 301 Teile das Reaktionssignal der dritten Teilfläche. Die jeweils anderen Signalkomponenten beeinflussen die Ergebnisse nicht. Der Unterschied besteht nur darin, daß einmal das 300fache, einmal das 299fache und einmal das 301fache der Einzelreaktion berechnet wird. Diese Abweichung ist aber durch eine einfache Umrechnung mit den entsprechenden Koeffizienten zu korrigieren.
Die Abweichungen von der Nennfrequenz 30 Hz betragen +/-0,33% und sind somit praktisch ohne Einfluß auf das Ergebnis.
Das Verfahren ist aber nur dann anwendbar, wenn in dem betrachteten Zeitabschnitt exakt vollständige Schwingungen verwendet werden. Diese Bedingung wird dann eingehalten, wenn alle verwendeten Schwingungen aus einem Grundraster abgeleitet werden.

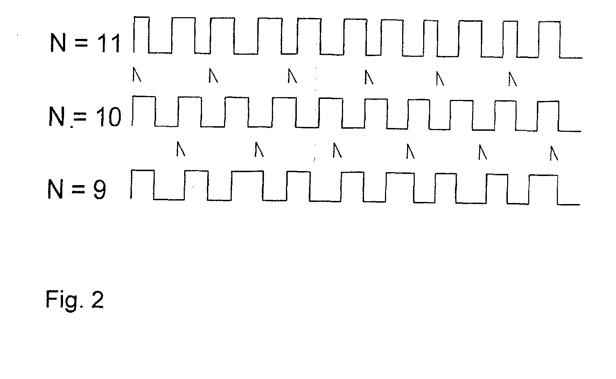

Fig. 2. zeigt ein Zeitdiagramm für die Erzeugung der Schwingungen. In diesem Beispiel werden aus einem Grundraster mit 60 Takten Rechteckschwingungen abgeleitet. Die in der Mitte dargestellte Kurve hat die Nennschwingungszahl N = 10, d.h. jede Schwingung enthält 6 Takte. Durch das Verkürzen um einzelne Takte an den sechs markierten Stellen erhält man die oben dargestellte Kurve mit 11 Schwingungen. Werden die Schwingungen an den sechs markierten Stellen um jeweils einen Takt verlängert, indem der zu diesem Zeitpunkt vorhandene Zustand um einen Takt verlängert wird, dann ergeben sich in dem gleichen Zeitraum N = 9 Schwingungen. Betrachtet man immer vollständige Schwingungen, dann kann man den Signalverlauf auch durch die Angabe der Schwingungslänge beschreiben, wenn man immer den Verlauf Hochpegel - Tiefpegel als eine Schwingung bezeichnet.

N = 11  besteht aus 6 Schwingungen zu 5 Takten (3 mal 2+3 Takte, 3 mal 3+2 Takte) und 5 Schwingungen zu 6 Takten.
N = 10  besteht aus 10 Schwingungen zu 6 Takten.
N = 9  besteht aus 6 Schwingungen zu 7 Takten (3 mal 4+3 Takte, 3 mal 3+4 Takte) und 3 Schwingungen zu 6 Takten.

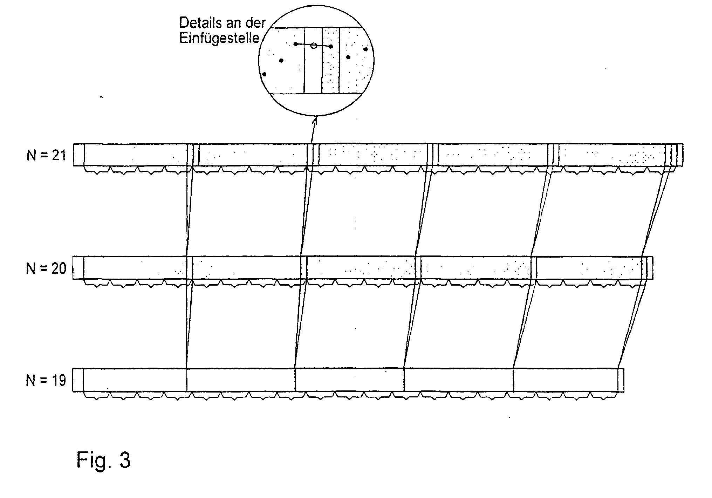

Selbstverständlich sind bei einem so groben Taktraster die Unterschiede zwischen den jeweils zwei Schwingungslängen deutlich zu erkennen. Benutzt man mehr Takte je Schwingung und eine größere Anzahl von Schwingungen, dann sind die Unterschiede auch kleiner, und man kann alle Schwingungen als nahezu kontinuierlich betrachten.
Das Prinzip der zyklischen Addition ist in Fig. 3 dargestellt.
Hier ist ein Beispiel mit N = 20 Schwingungen zu je 5 Takten gewählt. N = 20 symbolisiert den Signalverlauf, der als Summensignal über den Weg Elektrode - Verstärker - Analog-Digital-Wandler eingelesen wurde. Symbolisch ist hier dargestellt, das vor dem schraffiert gezeichneten eigentlichen Signalteil ein Einschwingbereich vorhanden ist und am Ende wenigstens eine Schwingung angehängt ist, um das letzte Reaktionssignal noch fehlerfrei auswerten zu können. Die Zykluslängen mit n = 5 Takten für die zyklische Summation ist unterhalb des schraffierten Teiles angegeben. Mit dieser Summation erhält man nur das Signal, das mit N = 20 stimuliert wurde.
Fig. 3 zeigt oben mit der Bezeichnung N = 21 symbolisch den Signalverlauf, der durch das Einfügen von Takten an den Stellen entsteht, die bei der Erzeugung der Schwingungen um einen Takt verkürzt wurden. Vergrößert ist hier dargestellt, wie der Signalpegel für den eingefügten Takt als Mittelwert aus dem vorhergehenden und dem nachfolgenden Signalpegel gebildet wird. Durch das Einfügen der Takte entsteht die längere Signalfolge mit N = 21 Schwingungen zu je 5 Takten. Wird die zyklische Summation hier auch mit N = 21 Zyklen zu n = 5 Takten durchgeführt, so erhält man nur das Signal, das mit N = 21 stimuliert wurde.

Fig. 3 zeigt unten mit der Bezeichnung N = 19 symbolisch den Signalverlauf, der durch das Entfernen von Takten an den Stellen entsteht, an denen bei der Erzeugung die Schwingungen um einen Takt verlängert wurden. Durch das Entfernen der Takte entsteht die kürzere Signalfolge mit N = 19 Schwingungen zu je 5 Takten. Wird die zyklische Summation hier auch mit N = 19 Zyklen zu n = 5 Takten durchgeführt, so erhält man nur das Signal, das mit N = 19 stimuliert wurde.

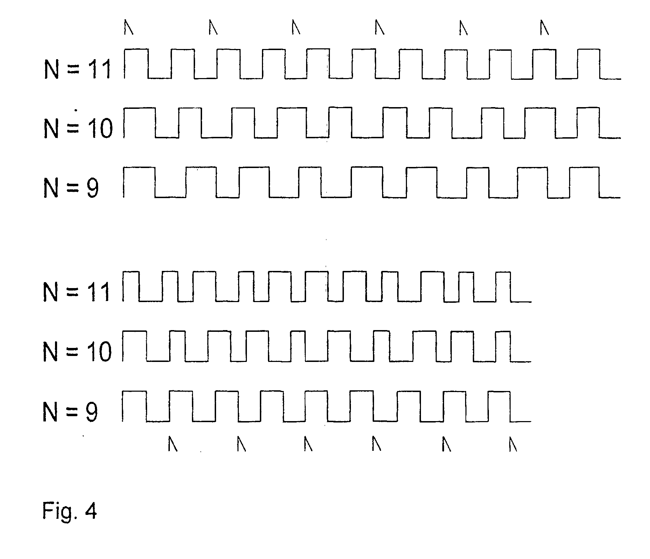

Fig. 4 zeigt ein Zeitdiagramm für die zyklische Addition, wobei die gleichen Stimulierungssignale, wie in Fig. 2 verwendet wurden, also ein Signal mit N = 10 Schwingungen und n = 6 Takten, aus dem auch Signale mit N = 11 und N = 9 Schwingungen abgeleitet wurden. Die Umwandlung der Signale erfolgt nach dem gleichen Prinzip, wie in Fig. 3 dargestellt. Im oberen Teil der Fig. 4 werden für die Rückgewinnung des Signals N = 11 an den Stellen Takte eingefügt, an denen bei der Erzeugung die Schwingung um einen Takt verkürzt wurden und im unteren Teil der Fig. 4 werden für die Rückgewinnung des Signals N = 9 dort Takte entfernt, wo bei der Erzeugung die Schwingung um einen Takt verlängert wurde. Die Stellen, an denen die Veränderungen der Taktfolge vorgenommen wurden, sind eingezeichnet. Setzt man Reaktionssignal gleich Stimulationssignal, dann stellen die dargestellten Signalverläufe die Komponenten des Reaktionssignals dar. Im oberen Teil ergibt sich die Kurve mit N = 11 und im unteren Teil die Kurve mit N = 9 wieder mit einer Schwingung der Ausgangslänge n = 6. Das ist dann auch die Basis für die zyklische Addition.

An dieser Stelle ist darauf aufmerksam zu machen, daß bereits in Fig. 2 die Positionen für das Verkürzen der Schwingung um einen Takt um eine Schwingung gegenüber der Position für das Verlängern der Schwingung um einen Takt versetzt eingetragen sind. Ein solcher oder ähnlicher Versatz ist notwendig, um sicherzustellen, daß bei der Umwandlung für die zyklische Addition nicht die Teilabschnitte nochmals um einen Takt verlängert oder verkürzt werden, die beim Erzeugen der Schwingungen bereits schon einmal verlängert oder verkürzt wurden.

Für eine praktische Anwendung des vorgenannten Prinzips ist mit einer größeren Anzahl von Takten je Schwingung und mit einer größeren Anzahl von Schwingungen in dem für die Messung vorgegebenen Zeitraum zu arbeiten. Betrachtet man wieder eine Stimulierung mit einem 30 Hz Flicker Signal, dann sind folgende Werte sinnvoll: Taktfrequenz 2 kHz.

Um eine starre Kopplung von Stimulierung und Aufnahme des Reaktionssignals, d.h. Taktierung des Analog-Digital-Wandlers, zu erhalten, ist die Abtastrate 0,5 ms.

Eine Schwingung kann aus 66 oder 67 Takten gebildet werden. Vorteilhaft ist die Verwendung von 67 Takten, da 67 selbst eine Primzahl ist und somit gewährleistet wird, daß die Anzahl der Schwingungen teilerfremd zu 67 ist und damit die Verlängerung oder Verkürzung um einen Takt im Gesamtablauf gleichmäßig über alle Takte der Schwingung verteilt sind.

Dadurch tritt der Fehler durch das Entfernen oder Einfügen von Takten an allen Stellen der Schwingung gleich oft auf, und der Fehler eliminiert sich praktisch vollständig. Die Veränderung um einen Takt ist bei 67 Takten eine Abweichung um 1,5% vom Nennwert, demzufolge entspricht eine Veränderung um zwei Takte einer Abweichung von 3% vom Nennwert. Will man bei 67 Takten eine symmetrische Schwingung erzeugen, dann muß die Hälfte der Schwingungen mit einem Tastverhältnis High:Low = 33:34 und die andere Hälfte mit einem Tastverhältnis High:Low = 34:33 betrieben werden.

Benutzt man eine Meßdauer von 35 Sekunden, dann ergeben sich bei 30 Hz 1050 Schwingungen. Bei einer Abweichung von 3% vom Nennwert lassen sich damit 61 verschiedene Schwingungen erzeugen, d.h. es lassen sich 61 verschiedene Teilflächen ansteuern.

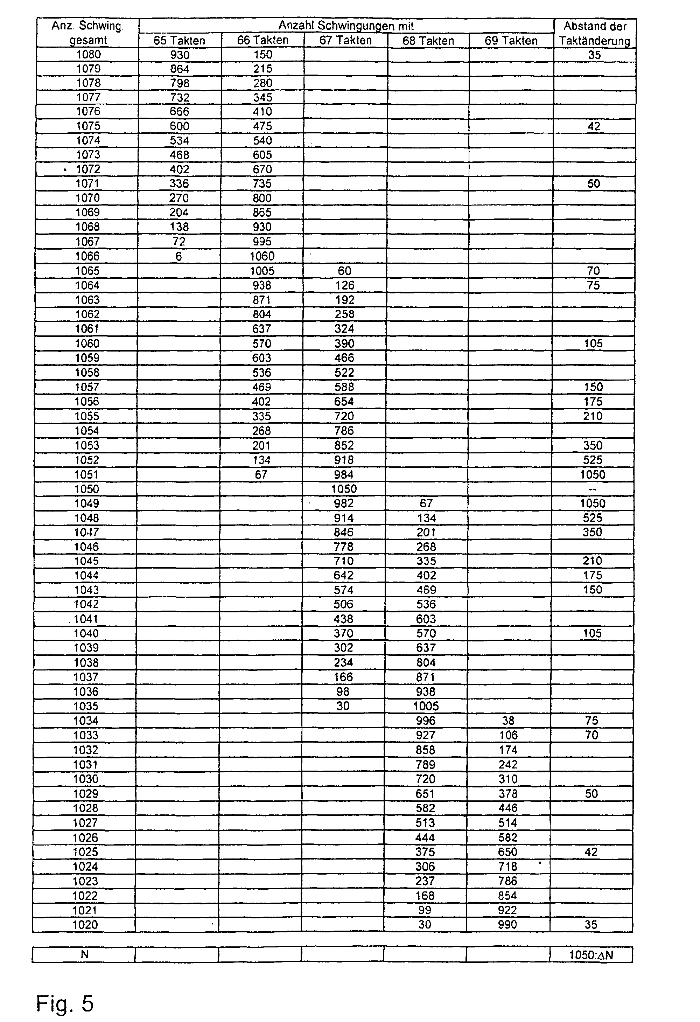

Fig. 5 zeigt eine Tabelle mit praktischen Werten der Schwingungen bei den genannten Bedingungen. Es ist zu erkennen, daß bei N = 1050 nur Schwingungen mit 67 Takten auftreten. Bei N = 1051 gibt es 67 Schwingungen mit 66 Takten, d.h. 67 mal wurde die Schwingung um je einen Takt verkürzt und es gibt 984 Schwingungen mit 67 Takten. Es werden also alle Schwingungen aus 70350 Takten abgeleitet. In der letzten Spalte ist der Abstand der auszublendenden oder einzublendenden Takte angegeben. Dieser Abstand ergibt sich für eine Schwingungszahl $N_i$ aus:

$$\text{Abstand} = \frac{1050}{|N_i\text{-}1050|}$$

Für die Fälle, bei denen sich kein ganzzahliger Wert für den Abstand ergibt, ergeben sich die Punkte aus dem gerundeten Wert des vielfachen Abstandes. Somit sind alle Schwingungszahlen zwischen 1020 und 1080 ableitbar. In der Tabelle sind nur die ganzzahligen Werte des Abstandes eingetragen.

Mit einer größeren Anzahl von Schwingungen lassen sich auch bei gleicher zulässiger Abweichung vom Nennwert der Schwingung noch mehr unterschiedliche Schwingungen erzeugen. Wenn man davon ausgeht, daß die 3% Grenze nicht überschritten wird, dann lassen sich mit 1750 Schwingungen, d.h. einer Meßdauer von etwa 58,34 Sekunden 103 verschiedene Schwingungen ableiten, d.h. es lassen sich 103 Teilflächen ansteuern.

Bedingung für die Anwendung dieses Verfahrens ist, daß sich der Zustand des Stimulators, also des dargestellten Bildes, in einem Abstand von 0,5 msec verändern kann. Dabei müssen alle Teile des Bildes taktgenau umgeschaltet werden. Das ist nur mit einer mit LED bestückten Fläche möglich. Hier kann mit verschiedenen Varianten gearbeitet werden:

- Die LED werden so angeordnet und zusammengeschaltet, wie die Teilflächen dargestellt werden sollen. Das ist zwar einfacher, aber dafür nicht flexibel.

- Jedes LED-Element wird getrennt angesteuert, und durch das Zusammenwirken benachbarter Elemente werden Teilflächen gebildet. Das ist zwar aufwendiger, dafür aber flexibel.

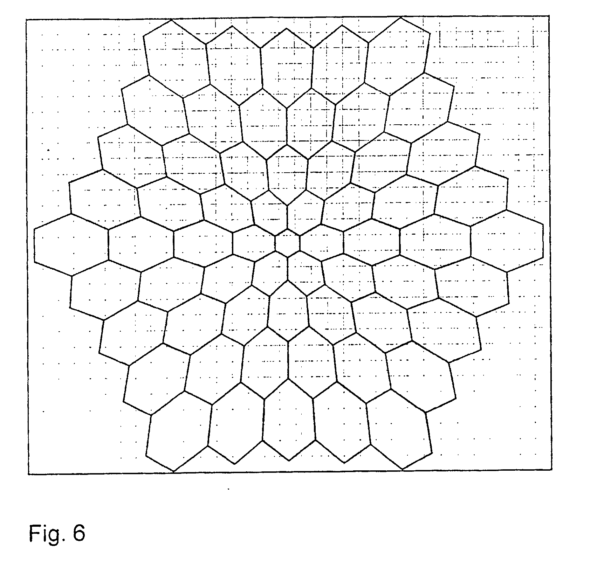

Fig. 6 zeigt, wie sich ein Bild mit 61 Sechsecken auf einer mit 33 x 29 = 957 LED bestückten Fläche darstellen läßt. Durch Bewertung der zugehörigen Flächenanteile der LED zu den Teilflächen des Bildes lassen sich die Zuordnungen der LED zu den Teilflächen berechnen.

Stimulatoren mit einer seriellen Bilddarstellung wie bei einem Bildschirm, einem Laserscanner oder einem LCD-Bildschirm sind für dieses Verfahren nicht geeignet.

Mit diesem Verfahren ergibt sich der folgende Ablauf für ein Beispiel, bei dem das Stimulierungsbild aus 61 Sechsecken besteht und die Stimulierung mit einem 30 Hz Flickersignal erfolgt.

In der Steuereinheit 3, die in Fig. 1 dargestellt ist, wird eine Taktfolge mit 2 kHz erzeugt, aus der das 30 Hz Signal mit 67 Takten je Schwingung gebildet wird. Diese Taktfolge besteht aus drei aufeinanderfolgenden Teilen:

- Einem ersten Teil, aus dem eine Schwingung abgeleitet wird, die zum Herstellen des eingeschwungenen Zustandes und zur Adaption des Auges dient. Dieser Teil enthält zum Beispiel 15 Schwingungen zu 30 Hz.
- Einem zweiten Teil, der für die Messung verwendet wird. Dieser Teil enthält 1050 Schwingungen bei der mittleren Frequenz von 30 Hz und besteht aus 67 x 1050 = 70350 Takten.
- Einem dritten Teil, in dem mindestens noch eine Schwingung erzeugt wird und der dazu dient, die letzte Schwingung der Messung noch fehlerfrei zu erfassen.

Zum Abzählen der 30 Hz Schwingung werden nun 67 Takte für jede mittlere Schwingung verwendet. Davon werden für ein Tastverhältnis von 1:1 die Hälfte der Schwingungen mit 34 Takten High und 33 Takten Low und die andere Hälfte der Schwingungen mit 33 Takten High und 34 Takten Low erzeugt.

Die so erzeugten Schwingungen dienen im ersten und im dritten Teil der Taktfolge als Stimulierungssignal für alle Teilflächen. Im zweiten Teil wird die so erzeugte Schwingung nur für eine Teilfläche verwendet. Für eine zweite Teilfläche wird im Abstand von 1050 : 1 = 1050 Takten jeweils ein Takt weniger verwendet. Das heißt, die erste Schwingung wird um den ersten Takt verkürzt und besteht nur aus 66 Takten. Die nächste Verkürzung einer Schwingung erfolgt beim Takt Nummer 1051, dann beim Takt Nummer 2101 und so fort. Damit entstehen in der Taktfolge von 70350 Takten 1051 Schwingungen, die im Abstand von 1050 Takten kleine Sprünge mit einem Fehler von 1,5% enthalten. Diese Sprünge entstehen aber über die Gesamtfolge 67 mal und dabei immer an einer anderen Stelle der Schwingung, da 67 eine Primzahl ist. Der Gesamtfehler beträgt hier den 1050ten Teil von 1,5% und ist somit vernachlässigbar. Das Prinzip zeigt Fig. 2 für ein stark vereinfachtes Beispiel mit 11 Schwingungen, die aus jeweils 6 Takten bestehen.

Für eine zweite Teilfläche wird im Abstand von 1050 : 2 = 525 Takten ein Takt weniger verwendet. Damit entstehen in der Taktfolge 1052 Schwingungen. Für eine dritte Teilfläche beträgt der Abstand 1050 : 3 = 350 Takte und für eine vierte Teilfläche 1050 : 4 = 262,5 Takte. Berechnet man diesen Wert mit Rundung, so beträgt der Abstand alternierend 263 und 262 Takte. Auf diese Weise werden in der Taktfolge unterschiedliche Schwingungszahlen von 1051 bis 1080 Schwingungen erzeugt. Allgemein gilt für die Position A des einzufügenden Taktes:

$$A = A_0 + n(1050 : \Delta N)$$

Dabei ist $A_0$ die Position des ersten einzufügenden Taktes, $\Delta N$ ist die Abweichung der Schwingungszahl von 1050 und n ist die fortlaufende Nummer für den einzufügenden Takt, wobei der Klammerausdruck immer gerundet wird. Die zugehörigen Zahlenwerte sind in Fig. 5 als Tabelle dargestellt, wobei für den Abstand nur die ganzzahligen Werte eingetragen sind. Die Frequenz beträgt bei 1050 Schwingungen 30 Hz und steigt bis auf 30,857 Hz bei 1080 Schwingungen.

Mit dem gleichen Prinzip kann man durch Verlängern einer Schwingung um je einen Takt im Abstand von 1050 : 1 = 1050 Takten in der Taktfolge von 70350 Takten 1049 Schwingungen erzeugen. Dabei wird die erste Verlängerung bei Takt Nummer 35, die zweite bei Takt Nummer 1085 usw. durchgeführt. Dieses Prinzip ist in Fig. 2 für N = 9 dargestellt. Verwendet man einen Abstand von 1050 : 2 = 525 Takten, dann ergeben sich 1048 Schwingungen. Bei einem Abstand von 1050 : 30 = 35 Takten erhält man 1020 Schwingungen in der Taktfolge von 70350 Takten. Die zugehörigen Zahlenwerte sind in Fig. 5 angegeben.

Mit dieser Berechnung ergeben sich 61 verschiedene Folgen, die eine konstante Taktlänge von 70350 Takten haben. Jede dieser Folgen besteht aus einer ganzzahligen Anzahl von Schwingungen. Mit der Frequenz von 2 kHz wird der in Fig. 1 dargestellte Stimulator 2 gesteuert. Der Stimulator 2 besteht aus einem Feld von Leuchtdioden, mit dem das zur Stimulierung verwendete Bild erzeugt wird. Fig. 6 zeigt ein Beispiel, in dem Sechsecke als Teilflächen eingetragen sind, und die Zuordnung zu den Teilflächen erfolgt so, daß jede Leuchtdiode dem Sechseck zugeordnet wird, in dem der größte Teil seiner Fläche enthalten ist. Alle Leuchtdioden einer Teilfläche werden dabei mit der gleichen Signalfolge gesteuert und alle Dioden des gesamten Bildes werden mit dem gleichen Takt gesteuert.

Blickt der Patient nun auf dieses vom Stimulator erzeugte Bild, dann werden von den einzelnen Teilflächen der Netzhaut entsprechende Reaktionssignale erzeugt. Die Summe dieser Reaktionssignale werden als Summensignal mit einer Elektrode nicht-invasiv abgenommen verstärkt, gefiltert und einem Analog-Digital-Wandler zugeführt. Dieser Analog-Digital-Wandler wird mit dem gleichen Takt gesteuert, mit dem auch die Leuchtdioden gesteuert werden. Damit wird das abgenommene Signal in das gleiche Taktraster gebracht, wie es für die Erzeugung des Stimulationsbildes verwendet wurde.

In einem Beispiel 1 das nicht Teil der beanspruchte Erfindung ist, wird die Elektrode der Hornhaut des Auges plaziert.

Aus diesem Summensignal lassen sich nun die Reaktionssignale der einzelnen Teilflächen wiedergewinnen. Für das Reaktionssignal, dessen Teilfläche mit 1050 Schwingungen stimuliert wurde, wird das Summensignal zyklisch addiert und zwar mit einem Zyklus von 67 Takten. Damit ergibt sich das 1050fache des Reaktionssignals dieser Teilfläche. Alle anderen Reaktionssignale verschieben sich bei dieser Addition um eine oder mehrere Schwingungen und tragen somit nicht zu dem Ergebnis bei, da ein gleichspannungsfreies Signal immer gleichgroße Anteile im positiven und negativen Bereich hat.

Für die Rückgewinnung der Reaktionssignale der anderen Teilflächen wird die bei der Erzeugung des Stimulierungssignals vorgenommene Verzerrung wieder rückgängig gemacht. Das heißt, für die Rückgewinnung des Signals der mit 1051 Schwingungen stimulierten Teilfläche werden an den Stellen, an denen eine Verkürzung um einen Takt vorgenommen wurde, jetzt ein Takt eingefügt, dessen Wert aus dem Mittelwert des vorhergehenden und des nachfolgenden Abtastwertes besteht. Dadurch verlängert sich die Signalfolge auf 70350 + 67 Takte und somit auf 1051 Schwingungen zu je 67 Takten. Wendet man hierauf die zyklische Addition an, dann erhält man den 1051fachen Wert des betrachteten Reaktionssignals. Alle anderen Komponenten tragen nicht zum Ergebnis bei. Das Prinzip ist in Fig. 3 im oberen Teil für N = 21 dargestellt. Daß dabei wieder Signale entstehen, die der mittleren Stimulierungsfolge von 1050 Schwingungen entsprechen, zeigt Fig. 2 vereinfacht im oberen Teil, in dem für N = 11 die Takte eingefügt wurden. Analog wird für die Rückgewinnung der Reaktionssignale der anderen Teilflächen mit einer Schwingungszahl >1050 durch Einfügen von Takten verfahren.

Die Rückgewinnung des Signals mit 1049 Schwingungen erfolgt so, daß an den Stellen, an denen das Stimulierungssignal verlängert wurde, jetzt ein Takt entfernt wird. Man erhält eine Signalfolge, die aus 70350 - 67 Takten und somit aus 1049 Schwingungen zu 67 Takten besteht. Bei der zyklischen Addition entsteht das 1049fache des Reaktionssignals der betreffenden Teilfläche. Das Prinzip ist in Fig.3 für N = 19 dargestellt. Daß dabei wieder Signale entstehen, die der mittleren Stimulierungsfolge von 1050 Schwingungen entsprechen, zeigt Fig. 2 vereinfacht im unteren Teil, in dem für N = 9 die Takte entfernt wurden.

Die dabei entstehenden Sprünge entstehen über die gesamte Folge immer an einer anderen Stelle der 67 Takte und erzeugen somit praktisch keinen Fehler.

Die Auswertung der Meßergebnisse kann durch die Bestimmung der Amplitude und der Latenz des Spitzenwertes erfolgen. Weiterhin kann man mit Hilfe der Fouriertransformation die Signalkomponenten der Grundwelle und der Oberwelle und deren Phasenlage bestimmen. Dabei kann der Vorteil genutzt werden, daß man ein Stimulierungssignal verwendet, in dem bestimmte Signalkomponenten nicht oder nahezu nicht enthalten sind, wie z.B. bei einem Rechtecksignal mit dem Tastverhältnis 1:1, in dem die erste Oberwelle nicht enthalten ist. Damit ergeben sich günstige Bedingungen, um Rückschlüsse auf Nichtlinearitäten in dem Reaktionssignal zu ziehen.

Das Verfahren ist auch für alle andere Messungen vorteilhaft anwendbar, bei denen mit einer zyklischen Reizung und einem eingeschwungenen Zustand gearbeitet wird. Die Anzahl M der über die gesamte Meßdauer T parallel meßbaren Teilergebnisse bei einer zulässigen Toleranz $\Delta$ der Stimulierungsfrequenz f ist:

$$M \leq 1 + 2 \cdot \Delta \cdot f \cdot T$$

Aufstellung der Bezugszeichen

1 Patient

2 Stimulator

3 Steuereinheit

4 Anzeigeeinheit

5 Tastatur

6 Verstärker

7 Anolog-Digital-Wandler

**Patentansprüche**

1. Verfahren zur nicht-invasiven Bestimmung der Topographie für die Reaktionssignale eines Auges, insbesondere durch die gleichzeitige Messung der von mehreren Teilflächen der Netzhaut eines Auges durch Lichtreize erzeugten Reaktionssignale, die sich bei zyklischer Reizung im eingeschwungenen Zustand ergeben, **dadurch gekennzeichnet, daß** in einem vorgegebenen Zeitabschnitt für die Messung jede Teilfläche durch eine ganzzahlige Anzahl von vollständigen Schwingungen der Lichtreize stimuliert wird und daß sich die Anzahl der Schwingungen in dem vorgegebenen Zeitabschnitt für die einzelnen Teilflächen jeweils um ein ganzzahliges Vielfaches von 1 unterscheidet, indem in einem vorgegebenen Zeitabschnitt für die Messung eine Taktfolge mit einer bestimmten Gesamtanzahl von Takten gebildet wird, aus der eine ganzzahlige Anzahl von Zyklen mit jeweils einer Schwingung abgezählt wird und daß die Erzeugung der Schwingungen für die Teilflächen derart erfolgt, daß

   - für eine erste Teilfläche eine mittlere Anzahl von Schwingungen gebildet wird, indem die in der Taktfolge erzeugten Zyklen verwendet werden,
   - für eine erste Gruppe von Teilflächen eine erste Gruppe von Schwingungen gebildet wird, indem die erzeugten Zyklen in bestimmten und weitgehend konstanten Abständen um einzelne Takte verlängert werden, wodurch sich eine kleinere Anzahl von Schwingungen in der vorgegebenen Gesamtanzahl von Takten ergibt, daß für jede der dieser Gruppe zugeordneten Teilflächen ein anderer Abstand für die Verlängerung um einen Takt verwendet wird und daß die Anzahl der zur Verlängerung verwendeten Takte jeweils ein ganzzahliges Vielfaches der Anzahl der für die mittlere Anzahl von Schwingungen verwendeten Takte ist, und
   - für eine zweite Gruppe von Teilflächen eine zweite Gruppe von Schwingungen gebildet wird, indem die erzeugten Zyklen in bestimmten und weitgehend konstanten Abständen um einzelne Takte verkürzt werden, wodurch sich eine größere Anzahl von Schwingungen in der vorgegebenen Gesamtanzahl von Takten ergibt, daß für jede der dieser Gruppe zugeordneten Teilflächen ein anderer Abstand für die Verkürzung um einen Takt verwendet wird und die Anzahl der zur Verkürzung verwendeten Takte jeweils ein ganzzahliges Vielfaches der Anzahl der für die mittlere Schwingung verwendeten Takte ist, und

   daß für das Erreichen des eingeschwungenen Zustandes mehrere zusätzliche Schwingungen vor dem Zeitabschnitt für die Messung und für die Auswertung der letzten Schwingung wenigstens eine zusätzliche Schwingung nach dem Zeitabschnitt für die Messung vorgesehen ist, daß ein aus der Überlagerung der Reaktionssignale aller Teilflächen gebildetes Summensignal am Patienten nicht-invasiv abgenommen wird, das verstärkt und gefiltert einem Analog-Digital-Wandler zugeführt wird, der das Signal mit den gleichen, für die Erzeugung der Schwingungen verwendeten Takten übernimmt und daß die Reaktionssignale für die einzelnen Teilflächen durch zyklische Summation der aufeinanderfolgenden Teile des Summensignals zurückgewonnen werden, wobei für die Summation dieselben Zyklen verwendet werden, wie für die Schwingungen zur Stimulierung der entsprechenden Teilflächen.

2. Verfahren nach Anspruch 1, **dadurch gekennzeichnet, daß** für die zyklische Addition zur Rückgewinnung der Reaktionssignale für die einzelnen Teilflächen die Zyklen verwendet werden, wie sie für die Erzeugung der mittleren

EP 1 237 475 B1

Anzahl von Schwingungen für die Lichtreize verwendet wurden und daß für die Schwingungen, die um jeweils einen Takt verlängert wurden bei der zyklischen Addition ein Abtastwert an der Stelle entfernt wird, an der bei der Erzeugung die Schwingung um einen zusätzliche Takt verlängert wurde und für die Zyklen, bei denen die Schwingung um einen Takt verkürzt wurde, an dieser Stelle ein zusätzlicher Wert eingefügt wird, der dem Mittelwert aus dem vorhergehenden und den nachfolgenden Abtastwert entspricht.

3. Verfahren nach Anspruch 1 oder 2, **dadurch gekennzeichnet, daß** die Zyklen für die Erzeugung der Lichtreize Sinusschwingungen enthalten.

4. Verfahren nach Anspruch 1 oder 2, **dadurch gekennzeichnet, daß** die Zyklen für die Erzeugung der Lichtreize Rechteckschwingungen enthalten, deren Tastverhältnis einstellbar ist.

5. Verfahren nach einem der Ansprüche 1 bis 4, **dadurch gekennzeichnet, daß** zur Rückgewinnung der Grund- und Oberwellen der Reaktionssignale für die einzelnen Teilflächen die Fouriertransformation angewendet wird.

6. Verfahren nach einem der Ansprüche 1 bis 4, **dadurch gekennzeichnet, daß** das Reaktionssignal ein visuell evoziertes Potential ist, das mit einer oder mehreren Elektroden nicht-invasiv am Kopf des Patienten abgenommen wird.

7. Anordnung zur Durchführung des Verfahrens nach einem der Ansprüche 1 bis 6 zur Bestimmung der Topographie für die Reaktionssignale eines Auges, insbesondere durch die gleichzeitige Messung der von mehreren Teilflächen der Netzhaut eines Auges durch Lichtreize erzeugten Reaktionssignale, die sich bei zyklischer Reizung im eingeschwungenen Zustand ergeben, **gekennzeichnet durch**

- Mittel zur Erzeugung von Lichtreizen auf Teilflächen der Netzhaut des Auges, die **durch** Signale steuerbar sind,
- eine Steuereinheit (3) mit Mitteln zur Erzeugung der Signale für die Steuerung der Lichtreize, die in einem vorgegebenen Zeitabschnitt für alle Teilflächen eine ganzzahlige Anzahl von Schwingungen erzeugen, wobei sich die Anzahl der Schwingungen für jede Teilfläche in dem vorgegebenen Zeitabschnitt um ein ganzzahliges Vielfaches von 1 unterscheidet und die Schwingungen aus einer vorgegebenen Gesamtanzahl von Takten gebildet werden und mit Mitteln, um in konstanten oder nahezu konstanten Abständen für eine erste Gruppe von Schwingungen Takte einzublenden sowie für eine zweite Gruppe von Schwingungen Takte auszublenden und mit Mitteln zum Abzählen der einzublendenden sowie auszublendenden Takte, mit einem Analog-Digital-Umsetzer für die Übernahme des Summensignals, der mit dem Takt für die Erzeugung der Steuersignale getaktet wird und mit Mitteln zur zyklischen Addition des digitalisierten Summensignals, wobei jeweils an der Stelle ein Meßwert entfernt wird, an dem bei das Signal für die Steuerung der Lichtreize um einen Takt verlängert wurde, und jeweils an der Stelle ein Meßwert eingefügt wird, an dem bei das Signal für die Steuerung der Lichtreize um einen Takt Verkürzt wurde und Mitteln zur Speicherung der Meßergebnisse,
- Mittel, um ein **durch** die zyklische Stimulierung erzeugtes Summensignal an dem zu untersuchenden Patienten abzunehmen, dieses zu verstärken und zu filtern und dem Analog-Digital-Umsetzer in der Steuereinheit (3) zuzuführen und
- Mittel zur Bedienung, zur Überwachung und zur Auswertung der Meßergebnisse.

8. Anordnung nach Anspruch 7, **dadurch gekennzeichnet, daß** die Lichtreize auf der Netzhaut durch ein Feld mit Leuchtdioden erzeugt werden, deren Helligkeitsverlauf einzeln oder in Gruppen durch Signale steuerbar ist.

9. Anordnung nach Anspruch 7, **dadurch gekennzeichnet, daß** die Steuereinheit (3) einen Rechner enthält, der

- die Schwingungen für die Steuerung des Helligkeitsverlaufes der Teilflächen berechnet,
- die Steuersignale für die Steuerung des LED - Feldes zur Erzeugung der Lichtreize erzeugt,
- den Analog - Digital - Wandler steuert,
- das digitalisierte Summensignal speichert,
- die Reaktionssignale der Teilflächen durch zyklische Summation berechnet und speichert,
- die Ergebnisse darstellt und auswertet und über den die Bedienung der Anordnung und Einstellung der Parameter des Verstärkers erfolgt.

**Claims**

1.  Method for non-invasively determining the topography for the reaction signals of an eye, in particular by simultaneously measuring the reaction signals that are generated by a plurality of partial surfaces of the retina of an eye by light stimuli and that are produced in the steady state in the case of cyclic excitation, **characterized in that** each partial surface is stimulated by an integral number of complete oscillations of the light stimuli in a prescribed time interval for the measurement, and **in that** the number of the oscillations in the prescribed time interval for the individual partial surfaces in each case differs by an integral multiple of 1 by virtue of the fact that there is formed in a prescribed time interval for the measurement a clock pulse rate with a specific total number of clock pulses from which an integral number of cycles with one oscillation in each case is counted, and **in that** the generation of the oscillations for the partial surfaces is performed in such a way that

    -   a mean number of oscillations is formed for a first partial surface by using the cycles generated at the clock pulse rate,
    -   for a first group of partial surfaces a first group of oscillations is formed by prolonging the generated cycles at specific and largely constant intervals by individual clock pulses, as a result of which a smaller number of oscillations occurs in the prescribed total number of clock pulses, that for each of the partial surfaces assigned to this group a different interval is used for the prolongation by a clock pulse, and that the number of the clock pulses used for the prolongation is in each case an integral multiple of the number of the clock pulses used for the mean number of oscillations, and
    -   for a second group of partial surfaces a second group of oscillations is formed by shortening the generated cycles at specific and largely constant intervals by individual clock pulses, as a result of which a larger number of oscillations occurs in the prescribed total number of clock pulses, that for each of the partial surfaces assigned to this group a different interval is used for the shortening by a clock pulse, and the number of the clock pulses used for the shortening is in each case an integral multiple of the number of the clock pulses used for the mean oscillation, and

    **in that** a plurality of additional oscillations before the time interval for the measurement are provided for reaching the steady state, and at least one additional oscillation after the time interval for the measurement is provided for evaluating the last oscillation, **in that** an aggregate signal that is formed from the superimposition of the reaction signals of all the partial surfaces is tapped non-invasively at the patient and is fed, amplified and filtered, to an analogue-to-digital converter that accepts the signal with the same clock pulses used for generating the oscillations, and **in that** the reaction signals for the individual partial surfaces are recovered by cyclic summation of the successive portions of the aggregate signal, use being made for the summation of the same cycles as for the oscillations for stimulating the corresponding partial surfaces.

2.  Method according to Claim 1, **characterized in that** for the cyclic addition for recovering the reaction signals for the individual partial surfaces use is made of the cycles that were used to generate the mean number of oscillations for the light stimuli, and **in that**, during the cyclic addition, for the oscillations that were prolonged by one clock pulse in each case a sample is removed at the point at which the oscillation was prolonged by an additional clock pulse during the generation, and for the cycles during which the oscillation was shortened by one clock pulse an additional value is inserted at this point that corresponds to the mean value from the preceding and the subsequent sample.

3.  Method according to Claim 1 or 2, **characterized in that** the cycles for generating the light stimuli include sinusoidal oscillations.

4.  Method according to Claim 1 or 2, **characterized in that** the cycles for generating the light stimuli include square-wave oscillations whose pulse duty factor can be set.

5.  Method according to one of Claims 1 to 4, **characterized in that** the Fourier transformation is applied for recovering the fundamental and harmonic waves of the reaction signals for the individual partial surfaces.

6.  Method according to one of Claims 1 to 4, **characterized in that** the reaction signal is a visually evoked potential that is tapped non-invasively with one or more electrodes on the patient's head.

7.  System for carrying out the method as claimed in one of claims 1 to 6 for determining the topography for the reaction signals of an eye, in particular by simultaneously measuring the reaction signals that are generated by a plurality of partial surfaces of the retina of an eye by light stimuli and that are produced in the steady state in the case of cyclic excitation, **characterized by**

- means for generating light stimuli on partial surfaces of the retina of the eye, which can be controlled by signals,
- a control unit (3) having means for generating the signals for controlling the light stimuli that generate an integral number of oscillations in a prescribed time interval for all partial surfaces, the number of the oscillations for each partial surface differing in the prescribed time interval by an integral multiple of 1, and the oscillations being formed from a prescribed total number of clock pulses, and having means for fading in clock pulses at constant or virtually constant intervals for a first group of oscillations, and for fading out clock pulses for a second group of oscillations, and having means for counting the clock pulses to be faded in and faded out, having an analogue-to-digital converter for accepting the aggregate signal that is clocked with the clock pulse for generating the control signals, and having means for the cyclic addition of the digitized aggregate signal, there being removed in each case a measured value at the point at which there was prolongation by one clock pulse upon the signal for controlling the light stimuli, and there being inserted in each case a measured value at the point at which there was shortening by one clock pulse upon the signal for controlling the light stimuli, and means for storing the measurement results,
- means for tapping an aggregate signal, generated by the cyclic stimulation, at the patient to be examined, amplifying and filtering said signal and feeding it to the analogue-to-digital converter in the control unit (3), and
- means for handling, monitoring and evaluating the measurement results.

8. System according to Claim 7, **characterized in that** the light stimuli are generated on the retina by an array of light-emitting diodes whose brightness profile can be controlled individually or in groups by signals.

9. System according to Claim 7, **characterized in that** the control unit (3) includes a computer, that

- calculates the oscillations for controlling the brightness profile of the partial surfaces,
- generates the control signals for controlling the LED array for generating the light stimuli,
- controls the analogue-to-digital converter,
- stores the digitized aggregate signal,
- calculates and stores the reaction signals of the partial surfaces by cyclic summation, and
- displays and evaluates the results and via which the operation of the system and setting of the parameters of the amplifier are performed.

**Revendications**

1. Procédé de détermination non invasive de la topographie pour les signaux de réaction d'un oeil, notamment par la mesure simultanée des signaux de réaction provoqués par des excitations lumineuses de plusieurs surfaces partielles de la rétine d'un oeil et produits lors d'une excitation cyclique en régime permanent, **caractérisé en ce que** chaque surface partielle est stimulée pendant un intervalle de temps prédéfini pour la mesure par un nombre entier d'oscillations complètes des excitations lumineuses et que le nombre d'oscillations pendant l'intervalle de temps prédéfini pour chacune des surfaces partielles présente à chaque fois une différence égale à un multiple entier de 1 en formant, dans un intervalle de temps prédéfini pour la mesure, une série de périodes comprenant un nombre total donné de périodes de laquelle est déduit un nombre entier de cycles comprenant à chaque fois une oscillation et que les oscillations pour les surfaces partielles sont générées de telle manière que

- un nombre moyen d'oscillations est produit pour une première surface partielle en utilisant les cycles générés dans la série de périodes,
- un premier groupe d'oscillations est produit pour un premier groupe de surfaces partielles en prolongeant les cycles générés de quelques périodes à des écarts donnés et quasiconstants, ce qui résulte en un nombre inférieur d'oscillations pendant le nombre total donné de périodes, qu'un écart différent est utilisé pour chacune des surfaces partielles associées à ce groupe pour la prolongation d'une période et que le nombre de périodes utilisées pour la prolongation est à chaque fois un multiple entier du nombre de périodes utilisées pour le nombre moyen d'oscillations, et
- un deuxième groupe d'oscillations est produit pour un deuxième groupe de surfaces partielles en raccourcissant les cycles générés de quelques périodes à des écarts donnés et quasi constants, ce qui résulte en un nombre supérieur d'oscillations pendant le nombre total donné de périodes, qu'un écart différent est utilisé pour chacune des surfaces partielles associées à ce groupe pour le raccourcissement d'une période et le nombre de périodes utilisées pour le raccourcissement est à chaque fois un multiple entier du nombre de périodes utilisées pour le nombre moyen d'oscillations, et

que pour atteindre le régime permanent, plusieurs oscillations supplémentaires sont prévues avant l'intervalle de temps pour la mesure et au moins une oscillation supplémentaire est prévue après l'intervalle de temps pour la mesure pour l'évaluation de la dernière oscillation, qu'un signal total formé par la superposition des signaux de réaction de toutes les surfaces partielles est prélevé de manière non invasive sur le patient, lequel est amplifié et filtré pour ensuite être acheminé à un convertisseur analogique/numérique qui prend en charge le signal avec les mêmes périodes que celles utilisées pour la production des oscillations et que les signaux de réaction pour chacune des surfaces partielles sont reconstitués par totalisation cyclique des parties successives du signal total, les cycles utilisés pour la totalisation étant les mêmes que ceux utilisés pair les oscillations pour stimuler les surfaces partielles correspondantes.

2. Procédé selon la revendication 1, **caractérisé en ce que** les cycles utilisés pour l'addition cyclique destinée à reconstituer les signaux de réaction pour chacune des surfaces partielles sont les mêmes que ceux qui ont été utilisés pour la production du nombre moyen d'oscillations pour les excitations lumineuses et que pour les oscillations qui ont à chaque fois été prolongées d'une période, une valeur échantillonnée est retirée lors de l'addition cyclique à l'endroit auquel a eu lieu la prolongation d'une période supplémentaire lors de la production de l'oscillation et, pour les cycles lors desquels l'oscillation a été raccourcie d'une période, une valeur supplémentaire est insérée à l'endroit qui correspond à la valeur moyenne de la valeur échantillonnée précédente et suivante.

3. Procédé selon la revendication 1 ou 2, **caractérisé en ce que** les cycles de production des excitations lumineuses contiennent des oscillations sinusoïdales.

4. Procédé selon la revendication 1 ou 2, **caractérisé en ce que** les cycles de production des excitations lumineuses contiennent des oscillations rectangulaires dont le rapport cyclique est variable.

5. Procédé selon l'une des revendications 1 à 4, **caractérisé en ce que** la transformation de Fourier est utilisée pour la reconstitution des porteuses et des harmoniques des signaux de réaction pour chacune des surfaces partielles.

6. Procédé selon l'une des revendications 1 à 4, **caractérisé en ce que** le signal de réaction est un potentiel évoqué visuellement qui est prélevé de manière non invasive sur la tête du patient à l'aide d'une ou de plusieurs électrodes.

7. Arrangement pour la mise en oeuvre du procédé selon l'une des revendications 1 à 6 pour déterminer la topographie pour les signaux de réaction d'un oeil, notamment par la mesure simultanée des signaux de réaction provoqués par des excitations lumineuses de plusieurs surfaces partielles de la rétine d'un oeil et produits lors d'une excitation cyclique en régime permanent, **caractérisé par**

   - des moyens de production d'excitations lumineuses sur les surfaces partielles de la rétine de l'oeil, lesquels peuvent être commandés par des signaux,
   - un module de commande (3) comprenant des moyens pour générer les signaux de commande des excitations lumineuses qui génèrent un nombre entier d'oscillations pour toutes les surfaces partielles pendant un intervalle de temps prédéfini, le nombre d'oscillations pour chaque surface partielle présentant une différence égale à un multiple entier de 1 dans l'intervalle de temps prédéfini et les oscillations étant formées à partir d'un nombre total donné de périodes et comprenant des moyens pour insérer des périodes à des écarts constants ou quasi constants pour un premier groupe d'oscillations ainsi que pour supprimer des périodes pour un deuxième groupe d'oscillations et comprenant des moyens pour compter les périodes à insérer et à supprimer, comprenant un convertisseur analogique/numérique pour la prise en charge du signal total, lequel est cadencé au rythme de production des signaux de commande et comprenant des moyens pour l'addition cyclique du signal total numérisé, une valeur mesurée étant à chaque fois retirée à l'endroit auquel le signal de commande des excitations lumineuses a été prolongé d'une période et une valeur mesurée étant à chaque fois insérée à l'endroit auquel le signal de commande des excitations lumineuses a été raccourci d'une période et des moyens de mémorisation des résultats de la mesure,
   - des moyens pour prélever sur le patient à examiner un signal total produit par la stimulation cyclique, pour amplifier et filtrer celui-ci et pour l'acheminer au convertisseur analogique/numérique dans le module de commande (3) et
   - des moyens pour commander, pour contrôler et pour analyser les résultats de la mesure.

8. Arrangement selon la revendication 7, **caractérisé en ce que** les excitations lumineuses sont produites sur la rétine par un réseau de diodes électroluminescentes dont la courbe de luminosité peut être commandée individuellement ou par groupes à l'aide de signaux.

9. Arrangement selon la revendication 7, **caractérisé en ce que** le module de commande (3) contient un ordinateur qui

   - calcule les oscillations pour la commande de la courbe de luminosité des surfaces partielles,
   - produit les signaux de commande pour la commande du réseau de LED destiné à produire les excitations lumineuses,
   - commande le convertisseur analogique/numérique,
   - mémorise le signal total numérisé,
   - calcule les signaux de réaction des surfaces partielles par addition cyclique et les mémorise,
   - présente et analyse les résultats et par le biais duquel sont réalisés la commande de l'arrangement et le réglage des paramètres de l'amplificateur.

Fig. 1

Fig. 2

Details an der
Einfügestelle

N = 21

N = 20

N = 19

Fig. 3

Fig. 4

| Anz. Schwing. gesamt | Anzahl Schwingungen mit | | | | | Abstand der Taktänderung |
|---|---|---|---|---|---|---|
| | 65 Takten | 66 Takten | 67 Takten | 68 Takten | 69 Takten | |
| 1080 | 930 | 150 | | | | 35 |
| 1079 | 864 | 215 | | | | |
| 1078 | 798 | 280 | | | | |
| 1077 | 732 | 345 | | | | |
| 1076 | 666 | 410 | | | | |
| 1075 | 600 | 475 | | | | 42 |
| 1074 | 534 | 540 | | | | |
| 1073 | 468 | 605 | | | | |
| 1072 | 402 | 670 | | | | |
| 1071 | 336 | 735 | | | | 50 |
| 1070 | 270 | 800 | | | | |
| 1069 | 204 | 865 | | | | |
| 1068 | 138 | 930 | | | | |
| 1067 | 72 | 995 | | | | |
| 1066 | 6 | 1060 | | | | |
| 1065 | | 1005 | 60 | | | 70 |
| 1064 | | 938 | 126 | | | 75 |
| 1063 | | 871 | 192 | | | |
| 1062 | | 804 | 258 | | | |
| 1061 | | 637 | 324 | | | |
| 1060 | | 570 | 390 | | | 105 |
| 1059 | | 603 | 466 | | | |
| 1058 | | 536 | 522 | | | |
| 1057 | | 469 | 588 | | | 150 |
| 1056 | | 402 | 654 | | | 175 |
| 1055 | | 335 | 720 | | | 210 |
| 1054 | | 268 | 786 | | | |
| 1053 | | 201 | 852 | | | 350 |
| 1052 | | 134 | 918 | | | 525 |
| 1051 | | 67 | 984 | | | 1050 |
| 1050 | | | 1050 | | | -- |
| 1049 | | | 982 | 67 | | 1050 |
| 1048 | | | 914 | 134 | | 525 |
| 1047 | | | 846 | 201 | | 350 |
| 1046 | | | 778 | 268 | | |
| 1045 | | | 710 | 335 | | 210 |
| 1044 | | | 642 | 402 | | 175 |
| 1043 | | | 574 | 469 | | 150 |
| 1042 | | | 506 | 536 | | |
| 1041 | | | 438 | 603 | | |
| 1040 | | | 370 | 570 | | 105 |
| 1039 | | | 302 | 637 | | |
| 1038 | | | 234 | 804 | | |
| 1037 | | | 166 | 871 | | |
| 1036 | | | 98 | 938 | | |
| 1035 | | | 30 | 1005 | | |
| 1034 | | | | 996 | 38 | 75 |
| 1033 | | | | 927 | 106 | 70 |
| 1032 | | | | 858 | 174 | |
| 1031 | | | | 789 | 242 | |
| 1030 | | | | 720 | 310 | |
| 1029 | | | | 651 | 378 | 50 |
| 1028 | | | | 582 | 446 | |
| 1027 | | | | 513 | 514 | |
| 1026 | | | | 444 | 582 | |
| 1025 | | | | 375 | 650 | 42 |
| 1024 | | | | 306 | 718 | |
| 1023 | | | | 237 | 786 | |
| 1022 | | | | 168 | 854 | |
| 1021 | | | | 99 | 922 | |
| 1020 | | | | 30 | 990 | 35 |
| N | | | | | | 1050:ΔN |

Fig. 5

19

Fig. 6